# EUROPÄISCHE PATENTANMELDUNG

(11) **EP 0 873 746 A2**
(43) Veröffentlichungstag der Anmeldung: **28.10.1998**
(21) Anmeldenummer: 98106959.4
(22) Anmeldetag: 16.04.1998
(51) Int. Cl.: A61K 7/13

(54) **Mittel zum Färben von keratinhaltigen Fasern**

(30) Priorität: 24.04.1997 DE 19717223
(71) Anmelder: Henkel Kommanditgesellschaft auf Aktien, 40589 Düsseldorf-Holthausen (DE)
(72) Erfinder: Möller, Hinrich, Dr., Dipl.-Chem., 40789 Monheim (DE); Höffkes, Horst, Dr., Dipl.-Chem., 40595 Düsseldorf (DE); Meinigke, Bernd, Dr., Dipl.-Chem., 51399 Burscheid (DE)

(57) **Zusammenfassung**

Es wird ein Mittel zum Färben von keratinhaltigen Fasern, insbesondere menschlichen Haaren, beansprucht, enthaltend Aminovinylaldehyde und/oder -ketone oder deren Derivate an der Carbonylgruppe mit der Formel I oder deren physiologisch verträglichen Salze 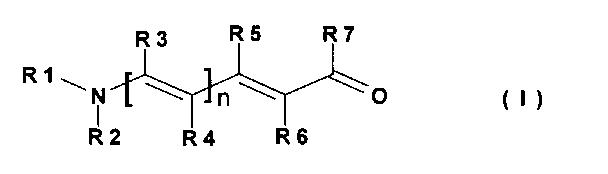
in der R¹ und R² unabhängig voneinander ein Wasserstoffatom, eine (C₁-C₄)-Alkylgruppe, Hydroxy-(C₁-C₄)-alkylgruppe, eine Phenylgruppe, die substituiert sein kann, oder gemeinsam mit dem N-Atom einen heterocyclischen Ring bilden, bedeuten,
R³, R⁴, R⁵, R⁶ und R⁷ für ein Wasserstoffatom, eine (C₁-C₄)-Alkylgruppe oder ein Halogenatom stehen und
n 0, 1 oder 2 bedeutet,
wobei R⁶ und R⁷ sowie R⁴ und R⁶, wenn n gleich 1 ist, jeweils gemeinsam einen Ring bilden können.

## Beschreibung

Die Erfindung betrifft ein Mittel zum Faden von keratinhaltigen Fasern, insbesondere menschlichen Haaren, das Aminovinylaldehyde und/oder -ketone oder deren Derivate an der Carbonylgruppe enthält, die Verwendung von Aminovinylaldehyden und/oder - ketonen oder Derivaten davon an der Carbonylgruppe als färbende Komponente in Haarfärbemitteln sowie ein Verfahren zum Färben von keratinhaltigen Fasern, insbesondere menschlichen Haaren.

Für das Färben von keratinhaltigen Fasern, z. B. Haaren, Wolle oder Pelzen, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind p-Phenylendiamin, p-Toluylendiamin, p-Aminophenol, o-Aminophenol, 1-(2 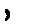 -Hydroxyethyl)-2,5-diaminobenzol, N,N-Bis-(2-hydroxy-ethyl)-p-phenylendiamin, 2-(2,5-Diaminophenoxy)-ethanol, 1-Phenyl-3-carboxyamido-4-amino-pyrazolon-5, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxyethylaminomethyl-4-amino-phenol, 4,4 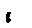 -Diaminodiphenylamin,.4-Amino-3-fluorphenol, 2-Aminomethyl-4-amino-phenol, 2-Hydroxymethyl-4-aminophenol, Bis-(2-hydroxy-5-aminophenyl)-methan, 1,4-Bis-(4-aminophenyl)-diazacycloheptan, 1,3-Bis(N(2-hydroxyethyl)-N(4-aminophenylamino))-2-propanol, 4-Amino-2-(2-hydroxyethoxy)-phenol sowie 4,5-Diaminopyrazol-Derivate nach EP 0 740 741 bzw. WO 94/08970 wie z.B. 4,5-Diamino-1-(2 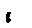 -hydroxyethyl)-pyrazol.

Als Kupplerkomponenten werden in der Regel m-Phenylendiaminderivate, Naphthole, Resorcin und Resorcinderivate, Pyrazolone und m-Aminophenole verwendet.

Spezielle Vertreter sind 1-Naphthol, Pyrogallol, 1,5-, 2,7- und 1,7-Dihydroxynaphthalin, o-Aminophenol, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 4-Chlorresorcin, 2-Chlor-6-methyl-3-aminophenol, 2-Methylresorcin, 5-Methylresorcin, 2,5-Dimethylresorcin, 2,6-Dihydroxypyridin, 2,6-Diaminopyridin, 2-Amino-3-hydroxypyridin, 2,6-Dihydroxy-3,4-diaminopyridin, 3-Amino-2-methylamino-6-methoxypyridin, 4-Amino-2-hydroxytoluol, 2,6-Bis-(2-hydroxyethylamino)-toluol, 2,4-Diaminophenoxyethanol, 1-Methoxy-2-amino-4-(2-hydroxyethylamino)-benzol, 2-Methyl-4-chlor-5-amino-phenol, 6-Methyl-1,2,3,4-tetrahydro-chinoxalin, 3,4-Methylendioxyphenol, 3,4-Methylendioxyanilin, 2,6-Dimethyl-3-amino-phenol, 3-Amino-6-methoxy-2-methylaminophenol, 2-Hydroxy-4-aminophenoxyethanol, 2-Methyl-5-(2-hydroxyethylamino)-phenol und 2,6-Dihydroxy-3,4-dimethylpyridin.

Bezüglich weiterer üblicher Farbstoffkomponenten wird ausdrücklich auf die Reihe 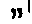 Dermatology 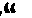 , herausgeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe), und Kap. 8, Seiten 264 - 267 (Oxidationsfarbstoffe), sowie das 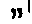 Europäische Inventar der Kosmetikrohstoffe 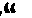 , 1996, herausgegeben von der Europäischen Kommission, erhältlich in Diskettenform vom Bundesverband der deutschen Industrie- und Handelsunternehmen für Arzneimittel, Reformwaren und Körperpflegemittel e.V., Mannheim, Bezug genommen.

Mit Oxidationsfarbstoffen lassen sich zwar intensive Färbungen mit guten Echtheitseigenschatten erzielen, die Entwicklung der Farbe geschieht jedoch i.a. unter dem Einfluß von Oxidationsmitteln wie z.B. H₂O₂, was in einigen Fällen Schädigungen der Faser zur Folge haben kann. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, daß die Färbungen häufig nur über unzureichende Echtheitseigenschaften verfügen.

Aus dem Stand der Technik ist die Verwendung von aromatischen Aldehyden bekannt.

Die deutsche Auslegeschrift DE 28 30 497 offenbart Haarfärbemittel, die Salicylaldehyd in Kombination mit Oxidationsfarbstoffvorprodukten enthalten, z.B. 2,5-Diaminotoluol, 4-Aminophenol oder 2,4-Diaminotoluol, die jedoch (in Kombination mit dem zusätzlich enthaltenen Wasserstoffperoxid) ein mehr oder weniger großes Sensibilisierungspotential aufweisen können. Die deutsche Auslegeschrift DE 29 32 489 offenbart ebenfalls H₂O₂-haltige Haarfärbemittel, die Benzaldehyde, z.B. 2-Hydroxy-3-methoxybenzaldehyd oder 4-Hydroxy-3-methoxybenzaldehyd, in Kombination mit Oxidationsfarbstoffvorprodukten enthalten. Die Patentschriften US 5,034,014 und US 5,199,954 beschreiben Haarfärberezeptur-Beispiele, die p-Dimethylaminobenzaldehyd oder p-Dimethylaminozimtaldehyd, z.B. in Kombination mit dem sensibilisierend wirkenden p-Phenylendiamin enthalten.

Die Patentschrift US 4,391,603 hat oxidationsmittelfreie Haarfärbemittel zum Gegenstand, die substituierte Benzaldehyde enthalten. Die dort offenbarten Haarfärbemittel stellen direktziehende Haarfärbemittel dar, mit denen sich nicht die Farbnuancen, Farbtiefen und Farbechtheiten von Oxidationshaarfarbstoffvorprodukthaltigen Haarfärbemitteln erreichen lassen.

Die Verwendung der unten näher beschriebenen Aminovinylaldehyde und -ketone oder Derivaten davon an der Carbonylgruppe zum Färben von keratinhaltigen Fasern ist bislang nicht bekannt.

Aufgabe der vorliegenden Erfindung ist es, Färbemittel für Keratinfasern, insbesondere menschliche Haare, bereitzustellen, die hinsichtlich der Farbtiefe, der Grauabdeckung und den Echtheitseigenschaffen qualitativ den üblichen Oxidationshaarfärbemitteln mindestens gleichwertig sind, ohne jedoch unbedingt auf Oxidationsmittel wie z.B. H₂O₂ angewiesen zu sein. Darüber hinaus dürfen die Färbemittel kein oder lediglich ein sehr geringes Sensibilisierungspotential aufweisen.

Überraschenderweise wurde nun gefunden, daß die in der Formel I dargestellten Aminovinylaldehyde und/oder -ketone oder deren Derivate an der Carbonylgruppe sich auch in Abwesenheit von oxidierenden Agenten hervorragend zum Färben von keratinhaltigen Fasern eignen. Sie ergeben Ausfärbungen mit hervorragender Brillanz und Farbtiefe und führen zu vielfältigen Farbnuancen. Der Einsatz von oxidierenden Agenten soll dabei jedoch nicht prinzipiell ausgeschlossen werden.

Gegenstand der Erfindung ist ein Mittel zum Färben von keratinhaltigen Fasern, insbesondere menschlichen Haaren, enthaltend Aminovinylaldehyde und/oder -ketone oder Derivate davon an der Carbonylgruppe mit der Formel I oder deren physiologisch verträglichen Salze 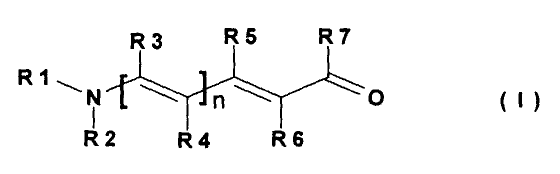
in der R¹ und R² unabhängig voneinander ein Wasserstoffatom, eine (C₁-C₄)-Alkylgruppe, Hydroxy-(C₁-C₄)-alkylgruppe, eine Phenylgruppe, die substituiert sein kann, oder gemeinsam mit dem N-Atom einen heterocyclischen Ring bilden, bedeuten,
R³, R⁴, R⁵, R⁶ und R⁷ für ein Wasserstoffatom, eine (C₁-C₄)-Alkylgruppe oder ein Halogenatom stehen und
n 0, 1 oder 2 bedeutet,
wobei R⁶ und R⁷ sowie R⁴ und R⁶, wenn n gleich 1 ist, jeweils gemeinsam einen Ring bilden können.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z.B. Baumwolle, Jute, Sisal, Leinen oder Seide, modifizierter Naturfasern, wie z.B. Regeneratcellulose, Nitro-, Alkyl- oder Hydroxyalkyl- oder Acetylcellulose und synthetischer Fasern, wie z.B. Polyamid-, Polyacrylnitril,-, Polyurethan- und Polyesterfasern verwendet werden.

Beispiele für Derivate an der Carbonylgruppe der Verbindungen mit der Formel I sind Iminoderivate sowie Ketale oder Acetale mit C₁-C₄-Alkylalkoholen.

Die erfindungsgemäß eingesetzten Aminovinylaldehyde und/oder -ketone oder deren Derivate an der Carbonylgruppe mit der Formel I sind vorzugsweise ausgewählt aus der Gruppe 3-Aminoacrolein, 3-Dimethylaminoacrolein, 4-Dimethylamino-3-buten-2-on, 5-Dimethylamino-2,4-pentadienal, 5-Diethylamino-2,4-pentadienal, 5-N-Methylanilino-2,4-pentadienal, 5-Pyrrolidino-2,4-pentadienal, 5-Piperidino-2,4-pentadienal, 5-Morpholino-2,4-pentadienal, 5-Dimethylamino-2-methyl-2,4-pentadienal, 5-Dimethylamino-3-methyl-2,4-pentadienal, 5-Dimethylamino-4-methyl-2,4-pentadienal, 5-Dimethylamino-3,5-dimethyl-2,4-pentadienal, 5-Dimethylamino-3-chlor-2,4-pentadienal, 6-Dimethylamino-3,5-hexadien-2-on 7-Dimethylamino-2,4,6-heptatrienal, 7-Morpholino-2,4,6-heptatrienal, 3-Dimethylaminomethylen-2-chlor-1-formyl-1-cyclohexen, 3-Dimethylaminomethylen-1-formyl-1-cyclopenten und deren physiologisch verträglichen Salzen. Als Salze sind insbesondere Chloride, Bromide, Perchlorate, Sulfate, Sulfonate, Methylsulfonate, Ethylsulfonate, Trifluormethansulfonate, Benzolsulfonate oder Toluolsulfonate zu nennen, wobei als Kationen die Alkalikationen bevorzugt sind. Beispiele für geeignete Derivate sind die Imionoderivate, die Acetale und Ketale. Beispiele für Iminoderivate sind N-(5-N 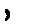 -Methylanilino-2,4-pentadienyliden-1-al)-N-methylaniliniumbromid und N-(5-Anilino-2,4-pentadienyliden)-anilin + HCl, Dimethyl-(7-chlor-2,4,6-heptatrienyliden)-ammoniumperchlorat und Dimethyl-(7-dimethylamino-2,4,6-heptatrienyliden)-ammoniumperchlorat.

Diese Substanzen sind literaturbekannt oder im Handel erhältlich.

Die voranstehend genannten Aminovinylaldehyde und -ketone oder deren Derivate an der Carbonylgruppe mit der Formel I werden vorzugsweise in den erfindungsgemäßen Mitteln in einer Menge von 0,03 bis 65 mmol, insbesondere von 1 bis 40 mmol, bezogen auf 100 g des gesamten Färbemittels, verwendet. Sie können als direktziehende Färbemittel oder in Gegenwart von Oxidationsfarbstoffvorprodukten eingesetzt werden.

Färbemittel, die als färbende Komponente die Aminovinylaldehyde und/oder -ketone oder deren Derivate an der Carbonylgruppe mit der Formel I allein enthalten, werden bevorzugt für Färbungen im Gelbbereich eingesetzt. Färbungen mit noch erhöhter Brillanz und verbesserten Echtheitseigenschatten (Lichtechtheit, Waschechtheit, Reibechtheit), vor allem im Gelb-, Orange-, Braun- und Schwarzbereich werden erzielt, wenn die erfindungsgemäß eingesetzten Verbindungen mit der Formel I gemeinsam mit mindestens einer weiteren Komponente (im folgenden Komponente B genannt), ausgewählt aus Verbindungen mit primärer oder sekundärer Aminogruppe oder Hydroxygruppe, ausgewählt aus primären oder sekundären aliphatischen oder aromatischen Aminen, stickstoffhaltigen heterocyclischen Verbindungen, Aminosäuren, aus 2 bis 9 Aminosäuren aufgebauten Oligopeptiden und aromatischen Hydroxyverbindungen, und/oder CH-aktiven Verbindungen, verwendet werden. Dies sind einerseits Verbindungen, die für sich alleine keratinhaltige Fasern nur schwach färben und erst gemeinsam mit den Aldehyden und/oder Ketonen der Formel I brillante Färbungen ergeben. Andererseits sind darunter aber auch Verbindungen, die bereits als Oxidationsfarbstoffvorprodukte eingesetzt werden.

Die voranstehend genannten Verbindungen der Komponente B können in einer Menge von jeweils 0,03 bis 65, insbesondere 1 bis 40 mmol, jeweils bezogen auf 100 g des gesamten Färbemittels, eingesetzt werden.

In allen Färbemitteln können auch mehrere verschiedene Aminovinylaldehyde und/oder -ke- tone der Formel I gemeinsam zum Einsatz kommen; ebenso können auch mehrere verschiedene Verbindungen der Komponente B gemeinsam verwendet werden. Unter diese Ausführungsform fällt auch die Verwendung von solchen Substanzen, die Reaktionsprodukte von Isatinderivaten der Formel I mit den genannten Verbindungen der Komponente B darstellen.

Geeignete Verbindungen mit primärer oder sekundärer Aminogruppe als Komponente B sind z.B. primäre aromatische Amine wie N,N-Dimethyl-, N,N-Diethyl-, N-(2-Hydroxyethyl)-N-ethyl-, N,N-Bis-(2-hydroxyethyl)-, N-(2-Methoxyethyl-), 2,3-, 2,4-, 2,5-Dichlor-p-phenylendiamin, 2-Chlor-p-phenylendiamin, 2,5-Dihydroxy-4-morpholinoanilin-dihydrobromid, 2-, 3-, 4-Aminophenol, 2-Aminomethyl-4-aminophenol, 2-Hydroxymethyl-4-aminophenol, o-, p-Phenylendiamin, o-Toluylendiamin, 2,5-Diaminotoluol, -phenol, -phenethol, 4-Amino-3-methylphenol, 2-(2,5-Diaminophenyl)-ethanol, 2,4-Diaminophenoxyethanol, 2-(2,5-Diaminophenoxy)-ethanol, 4-Methylamino-, 3-Amino-4-(2 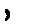 -hydroxyethyloxy)-, 3,4-Methylendiamino-, 3,4-Methylendioxyanilin, 3-Amino-2,4-dichlor-, 4-Methylamino-, 2-Methyl-5-amino-, 3-Methyl-4-amino-, 2-Methyl-5-(2-hydroxyethylamino)-, 6-Methyl-3-amino-2-chlor, 2-Methyl-5-amino-4-chlor-, 3,4-Methylendioxy-, 5-(2-Hydroxyethylamino)-4-methoxy-2-methyl-, 4-Amino-2-hydroxymethylphenol, 1,3-Diamino-2,4-dimethoxybenzol, 2-, 3-, 4-Aminobenzoesäure, -phenylessigsäure, 2,3-, 2,4-, 2,5-, 3,4-, 3,5-Diaminobenzoesäure, 4-, 5-Aminosalicylsäure, 3-Amino-4-hydroxy-, 4-Amino-3-hydroxy-benzoesäure, 2-, 3-, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 3,5-Diamino-4-hydroxybrenzcatechin, aromatische Aniline bzw. Phenole mit einem weiteren aromatischen Rest, wie sie in der Formel II dargestellt sind 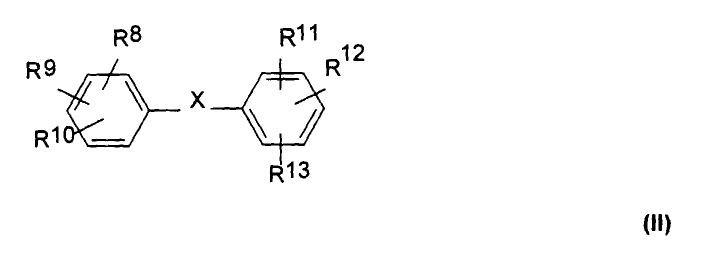
in der R⁸ für eine Hydroxy- oder eine Aminogruppe, die durch C₁₋₄-Alkyl-, C₁₋₄-Hydroxyalkyl-, C₁₋₄-Alkoxy- oder C₁₋₄-Alkoxy-C₁₋₄-alkylgruppen substituiert sein kann, steht,
R⁹, R¹⁰, R¹¹, R¹² und R¹³ für Wasserstoff, eine Hydroxy- oder eine Aminogruppe, die durch
C₁₋₄-Alkyl-, C₁₋₄-Hydroxyalkyl, C₁₋₄-Alkoxy-, C₁₋₄-Aminoalkyl- oder C₁₋₄-Alkoxy-C₁₋₄-alkylgruppen substituiert sein kann, stehen, und
X für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfonyl- oder Iminogruppe, ein Sauerstoff- oder Schwefelatom, oder eine Gruppe mit der Formel III 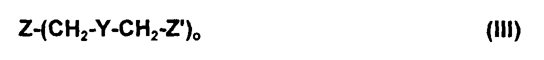
in der Y eine direkte Bindung, eine CH₂- oder CHOH-Gruppe bedeutet, Z und Z' unabhängig voneinander für ein Sauerstoffatom, eine NR¹⁴-Gruppe, worin R¹⁴ Wasserstoff, eine C₁₋₄-Alkyl- oder eine Hydroxy-C₁₋₄-alkylgruppe bedeutet, die Gruppe O-(CH₂)ₚ-NH oder NH-(CH₂)ₚ 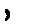 -O, worin p und p' 2 oder 3 sind, stehen und
o eine Zahl von 1 bis 4 bedeutet,
wie beispielsweise 4,4'-Diaminostilben, 4,4'-Diaminostilben-2,2'-disulfonsäure-mono- oder -di-Na-Salz, 4-Amino-4'-dimethylaminostilben, 4,4'-Diaminodiphenylmethan, - sulfid, -sulfoxid, -amin, 4,4'-Diaminodephenylamin-2-sulfonsäure, 4,4'-Diaminobenzophenon, -diphenylether, 3,3',4,4'-Tetraaminodiphenyl, 3,3',4,4'-Tetraaminobenzophenon, 1,3-Bis-(2,4-diaminophenoxy)-propan, 1,8-Bis-(2,5-diaminophenoxy)-3,6-dioxaoctan, 1,3-Bis-(4-aminophenylamino)propan, -2-propanol, 1,3-Bis-[N-(4-aminophenyl)-2-hydroxyethylamino]-2-propanol, N,N-Bis-[2-(4-aminophenoxy)-ethyl]-methylamin, N-Phenyl-1,4-phenylendiamin.

Die vorgenannten Verbindungen können sowohl in freier Form als auch in Form ihrer physiologisch verträglichen Salze, insbesondere als Salze anorganischer Säuren, wie Salz- oder Schwefelsäure, eingesetzt werden.

Geeignete stickstoffhaltige heterocyclische Verbindungen sind z.B. 2-, 3-, 4-Amino-, 2-Amino-3-hydroxy-, 2,6-Diamino-, 2,5-Diamino-, 2,3-Diamino-, 2-Dimethylamino-5-amino-, 2-Methylamino-3-amino-6-methoxy-, 2,3-Diamino-6-methoxy-, 2,6-Dimethoxy-3,5-diamino-, 2,4,5-Triamino-, 2,6-Dihydroxy-3,4-dimethylpyridin, 2,4-Dihydroxy-5,6-diamino-, 4,5,6-Triamino-, 4-Hydroxy-2,5,6-triamino-, 2-Hydroxy-4,5,6-triamino-, 2,4,5,6-Tetraamino-, 2-Methylamino-4,5,6-triamino-, 2,4-, 4,5-Diamino-, 2-Amino-4-methoxy-6-methyl-pyrimidin, 3,5-Diaminopyrazol, -1,2,4-triazol, 3-Amino-, 3-Amino-5-hydroxypyrazol, 2-, 3-, 8-Aminochinolin, 4-Aminochinaldin, 2-, 6-Aminonicotinsäure, 5-Aminoisochinolin, 5-, 6-Aminoindazol, 5-, 7-Aminobenzimidazol, -benzothiazol, 2,5-Dihydroxy-4-morpholinoanilin sowie Indol- und Indolinderivaten, wie 4-, 5-, 6-, 7-Aminoindol, 5,6-Dihydroxyindol, 5,6-Dihydroxyindolin und 4-Hydroxyindolin. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Geeignete aromatische Hydroxyverbindungen sind z.B. 2-, 4-, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-, 3-, 4-Methoxy-, 3-Dimethylamino-, 2-(2-Hydroxyethyl)-, 3,4-Methylendioxyphenol, 2,4-, 3,4-Dihydroxybenzoesäure, - phenylessigsäure, Gallussäure, 2,4,6-Trihydroxybenzoesäure, -acetophenon, 2-, 4-Chlorresorcin, 1-Naphthol, 1,5-, 2,3-, 2,7-Dihydroxynaphthalin, 6-Dimethylamino-4-hydroxy-2-naphthalinsulfonsäure, 3,6-Dihydroxy-2,7-naphthalinsulfonsäure.

Als CH-aktive Verbindungen können beispielhaft genannt werden 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, Fischersche Base (1,3,3-Trimethyl-2-methylenindolin), 2,3-Dimethyl-benzothiazoliumiodid, 2,3-Dimethyl-benzothiazolium-p-toluolsulfonat, Rhodanin, Rhodanin-3-essigsäure, 1-Ethyl-2-chinaldiniumiodid, 1-Methyl-2-chinaldiniumiodid Barbitursäure, Thiobarbitursäure, 1,3-Dimethylthiobarbitursäure, Diethylthiobarbitursäure, Oxindol, 3-Indoxylacetat, Cumaranon und 1-Methyl-3-phenyl-2-pyrazolinon.

Als Aminosäuren kommen bevorzugt alle natürlich vorkommenden und synthetischen α-Aminosäuren in Frage, z.B. die durch Hydrolyse aus pflanzlichen oder tierischen Proteinen, z.B. Kollagen, Keratin, Casein, Elastin, Sojaprotein, Weizengluten oder Mandelprotein, zugänglichen Aminosäuren. Dabei können sowohl sauer als auch alkalisch reagierende Aminosäuren eingesetzt werden. Bevorzugte Aminosäuren sind Arginin, Histidin, Tyrosin, Phenylalanin, DOPA (Dihydroxyphenylalanin), Ornithin, Lysin und Tryptophan. Aber auch andere Aminosäuren, wie z.B. 6-Aminocapronsäure, können eingesetzt werden.

Die Oligopeptide können dabei natürlich vorkommende oder synthetische Oligopeptide, aber auch die in Polypeptid- oder Proteinhydrolysaten enthaltenen Oligopeptide sein, sofern sie über eine für die Anwendung in den erfindungsgemäßen Färbemitteln ausreichende Wasserlöslichkeit verfügen. Als Beispiele sind z.B. Glutathion und die in den Hydrolysaten von Kollagen, Keratin, Gasen, Elastin, Sojaprotein, Weizengluten oder Mandelprotein enthaltenen Oligopeptide zu nennen. Bevorzugt sind dabei Mittel, die die Verbindungen mit der Formel I gemeinsam mit Verbindungen mit primärer oder sekundärer Aminogruppe oder mit aromatischen Hydroxyverbindungen enthalten.

Die Verbindungen der Komponente B werden besonders bevorzugt ausgewählt aus der Gruppe bestehend aus N-(2-Hydroxyethyl)-N-ethyl-, 2-Chlor-p-phenylendiamin, N,N-Bis(2-Hydroxyethyl)-p-phenylendiamin, 4-Aminophenol, p-Phenylendiamin, 2-(2,5-Diaminophenyl)-ethanol, 2,5-Diaminotoluol, 3,4-Methylendioxyanilin, 3-Amino-2,4-dichlor-, 2-Methyl-5-amino-, 3-Methyl-4-amino-, 2-Methyl-5-(2-hydroxyethylamino)-, 2-Methyl-5-amino-4-chlor-, 6-Methyl-3-amino-2-chlor-, 2-Aminomethyl-4-aminophenol, 2-Hydroxymethyl-4-aminophenol, 3,4-Methylendioxyphenol, 3,4-Diaminobenzoesäure, 2,5-Diamino-, 2-Dimethylamino-5-amino-, 3-Amino-2-methylamino-6-methoxy-, 2,3-Diamino-6-methoxy-, 3,5-Diamino-2,6-dimethoxy-, 2,6-Dihydroxy-3,4-dimethylpyridin, 2-Hydroxy-4,5,6-triamino-, 4-Hydroxy-2,5,6-triamino-, 2,4,5,6-Tetraamino-, 2-Methylamino-4,5,6-triamino-pyrimidin, 3,5-Diaminopyrazol, 3-Amino-5-hydroxypyrazol, 5,6-Dihydroxyindol und 5,6-Dihydroxyindolin sowie deren mit vorzugsweise anorganischen Säuren gebildeten physiologisch verträglichen Salze.

Auf die Anwesenheit von Oxidationsmitteln, z.B. H₂O₂, kann dabei verzichtet werden. Es kann jedoch u.U. wünschenswert sein, den erfindungsgemäßen Mitteln zur Erzielung der Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die Anwendungslösung, eingesetzt. Ein für menschliches Haar bevorzugtes Oxidationsmittel ist H₂O₂.

In einer bevorzugten Ausführungsform enthalten die erfindungsgemäßen Färbemittel zur weiteren Modifizierung der Farbnuancen neben den erfindungsgemäß enthaltenen Verbindungen zusätzlich übliche direktziehende Farbstoffe.

Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 4-Amino-2-nitrodiphenylamin-2 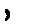 -carbonsäure, 6-Nitro-1,2,3,4-tetrahydrochinoxalin, Hydroxyethyl-2-nitro-toluidin, Pikraminsäure, 2-Amino-6-chloro-4-nitrophenol 4-Ethylamino-3-nitrobenzoesäure und 2-Chloro-6-ethylamino-1-hydroxy-4-nitrobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbe, Blauholz, Krappwurzel, Catechu, Sedre und Alkannawurzel enthalten.

Weitere in den erfindungsgemäßen Färbemitteln enthaltene Farbstoffkomponenten können auch Indole und Indoline, sowie deren physiologisch verträgliche Salze, sein. Bevorzugte Beispiele sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 6-Hydroxyindol, 6-Aminoindol und 4-Aminoindol. Weiterhin bevorzugt sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 6-Hydroxyindolin, 6-Aminoindolin und 4-Aminoindolin.

Es ist nicht erforderlich, daß die Oxidationsfarbstoffvorprodukte oder die fakultativ enthaltenen direktziehenden Farbstoffe jeweils einheitliche Verbindungen darstellen. Vielmehr können in den erfindungsgemäßen Färbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Färbemittel üblicherweise in einen wasserhaltigen kosmetischen Träger eingearbeitet werden. Geeignete wasserhaltige kosmetische Träger sind z.B. Cremes, Emulsionen, Gele oder auch tensidhaltige schäumende Lösungen wie z.B. Shampoos oder andere Zubereitungen, die für die Anwendung auf den keratinhaltigen Fasern geeignet sind. Falls erforderlich ist es auch möglich, die Färbemittel in wasserfreie Träger einzuarbeiten.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2 bis 15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumohlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Methylhydroxyalkyldialkoyloxyalkylammoniummethosulfate.

Ein Beispiel für ein als kationisches Tensid einsetzbares quaternäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyltrimethylammoniumchlorid/Acrylat-Copolymere und Octylacrylamid/Methylmethacrylat/tert.-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.-Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenertrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z.B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Für das Färbeergebnis kann es vorteilhaft sein, den Färbemitteln Ammonium- oder Metallsalze zuzugeben. Geeignete Metallsalze sind z.B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Erdalkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, - chlorid und -acetat bevorzugt sind. Diese Salze sind vorzugsweise in einer Menge von 0,03 bis 65, insbesondere von 1 bis 40, mmol bezogen auf 100 g des gesamten Färbemittels, enthalten.

Der pH-Wert der gebrauchsfertigen Färbezubereitungen liegt üblicherweise zwischen 2 und 11, vorzugsweise zwischen 5 und 9.

Ein weiterer Gegenstand der vorliegenden Erfindung betrifft die Verwendung von Aminovinylaldehyde und/oder -ketone oder deren Derivaten an der Carbonylgruppe mit der Formel I oder deren physiologisch verträglichen Salzen 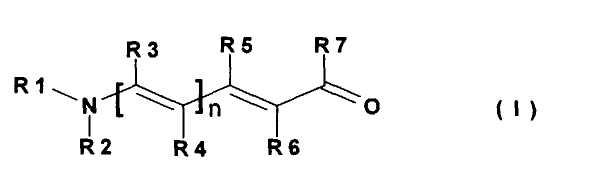
in der R¹ und R² unabhängig voneinander ein Wasserstoffatom, eine (C₁-C₄)-Alkylgruppe, Hydroxy-(C₁-C₄)-alkylgruppe, eine Phenylgruppe, die substituiert sein kann, oder gemeinsam mit dem N-Atom einen heterocyclischen Ring bilden, bedeuten,
R³, R⁴, R⁵, R⁶ und R⁷ für ein Wasserstoffatom, eine (C₁-C₄)-Alkylgruppe oder ein Halogenatom stehen und
n 0, 1 oder 2 bedeutet,
wobei R⁶ und R⁷ sowie R⁴ und R⁶, wenn n gleich 1 ist, jeweils gemeinsam einen Ring bilden können,
als eine färbende Komponente in Oxidationshaarfärbemitteln.

Noch ein weiterer Gegenstand der vorliegenden Erfindung betrifft ein Verfahren zum Färben von keratinhaltigen Fasern, insbesondere menschlichen Haaren, worin ein Färbemittel, enthaltend,
A) mindestens einen Aminovinylaldehyd oder ein -keton oder deren Derivate an der Carbonylgruppe mit der Formel I oder deren physiologisch verträglichen Salze 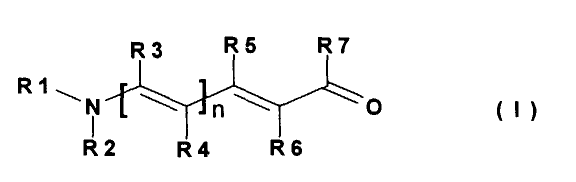
   in der R¹ und R² unabhängig voneinander ein Wasserstoffatom, eine (C₁-C₄)-Alkylgruppe, Hydroxy-(C₁-C₄)-alkylgruppe, eine Phenylgruppe, die substituiert sein kann, oder gemeinsam mit dem N-Atom einen heterocyclischen Ring bilden, bedeuten,
   R³, R⁴, R⁵, R⁶ und R⁷ für ein Wasserstoffatom, eine (C₁-C₄)-Alkylgruppe oder ein Halogenatom stehen und
   n 0, 1 oder 2 bedeutet,
   wobei R⁶ und R⁷ sowie R⁴ und R⁶, wenn n gleich 1 ist, jeweils gemeinsam einen Ring bilden können,
B) mindestens eine Verbindung mit primärer oder sekundärer Aminogruppe oder Hydroxygruppe, ausgewählt aus primären oder sekundären aliphatischen oder aromatischen Aminen, stickstoffhaltigen heterocyclischen Verbindungen, Aminosäuren, aus 2 bis 9 Aminosäuren aufgebauten Oligopeptiden und aromatischen Hydroxyverbindungen, und/oder mindestens eine CH-aktive Verbindung,
sowie übliche kosmetische Inhaltsstoffe auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

Die Aldehyde und/oder Ketone der Formel I und die Verbindungen der Komponente B können entweder gleichzeitig auf das Haar aufgebracht werden oder aber auch nacheinander, wobei es unerheblich ist, welche der beiden Komponenten zuerst aufgetragen wird. Die fakultativ enthaltenen Ammonium- oder Metallsalze können dabei der ersten oder der zweiten Komponente zingesetzt werden. Zwischen dem Auftragen der ersten und der zweiten Komponente können bis zu 30 Minuten Zeitabstand liegen. Auch eine Vorbehandlung der Fasern mit der Salzlösung ist möglich.

Die Aldehyde und/oder Ketone der Formel I und die Verbindungen der Komponente B können entweder getrennt oder zusammen gelagert werden, entweder in einer flüssigen bis pastösen Zubereitung (wäßrig oder wasserfrei) oder als trockenes Pulver. Werden die Komponenten in einer flüssigen Zubereitung zusammen gelagert, so sollte diese zur Verminderung einer Reaktion der Komponenten weitgehend wasserfrei sein. Bei der getrennten Lagerung werden die reaktiven Komponenten erst unmittelbar vor der Anwendung miteinander innig vermischt. Bei der trockenen Lagerung wird vor der Anwendung üblicherweise eine definierte Menge warmen (50 bis 80°C) Wassers hinzugefügt und eine homogene Mischung hergestellt.

### Beispiele

### Herstellung einer Färbelösung

Es wurde eine Aufschlämmung von 10 mMol eines Aminovinylaldehyds bzw. -ketons der Formel I, 10 mMol eines Oxidationsfarbstoffvorproduktes, 10 mMol Natriumacetat und einen Tropfen einer 20 %igen Fettalkylethersulfat-Lösung in 100 ml Wasser bereitet. Die Aufschlämmung wurde kurz auf ca. 80°C erhitzt und nach dem Abkühlen filtriert, der pH-Wert wurde anschließend auf 6 eingestellt.

In diese Färbelösung wurde bei 30°C 30 Minuten lang eine Strähne zu 90% ergrauten, nicht vorbehandelten Menschenhaares eingebracht. Die Strähne wurde dann 30 Sek. mit lauwarmem Wasser gespült, mit warmer Luft getrocknet und anschließend ausgekämmt.

Die jeweiligen Farbnuancen und Farbtiefen sind in der nachfolgenden Tabelle 1 wiedergegeben.

Die Farbtiefe wurde dabei nach folgender Skala bewertet:
- - :: keine oder eine sehr blasse Ausfärbung
- (+) :: schwache Intensität
- + :: mittlere Intensität
- +(+) :: mittlere bis starke Intensität
- ++ :: starke Intensität
- ++(+) :: starke bis sehr starke Intensität
- +++ :: sehr starke Intensität

**Tabelle 1**

| **Ausfärbungen mit 3-Dimethylaminoacrolein** | | |
|---|---|---|
| Komponente B | Färbenuance | Farbtiefe |
| 2,5-Diaminotoluol x H₂SO₄ | rotbraun | ++(+) |
| 2,4,5,6-Tetraaminopyrimidin x H₂SO₄ | orangegelb | ++ |
| 1,8-Bis-(2,5-diaminophenoxy)-3,6-dioxaoctan x 4HCl | dunkelbraun | ++(+) |
| 2-Methylamino-3-amino-6-methoxypyridin x 2HCl | dunkegelbbraun | ++(+) |
| 2-(2,5-Diaminophenyl)-ethanol x H₂SO₄ | orangerot | ++(+) |
| 2-Aminomethyl-4-aminophenol x 2HCl | gelb | ++(+) |
| N,N-Bis-(2-hydroxyethyl)-p-phenylendiamin x H₂SO₄ | orangerot | ++(+) |
| 4,4'-Diaminodiphenylamin x H₂SO₄ | schwarzbraun | +++ |
| 2,6-Dimethoxy-3,5-diaminopyridin x 2HCl | dunkelbraun | +++ |

**Tabelle 2**

| **Ausfärbungen mit 1-(2-Dimethylaminoethenyl)-5,5-dimethyl-cyclo-1-hexen-3-on** | | |
|---|---|---|
| Komponente B | Färbenuance | Farbtiefe |
| - | dunkelgelb | ++ |
| 2,5-Diaminotoluol x H₂SO₄ | dunkelbraun | ++ |
| 2,4,5,6-Tetraaminopyrimidin x H₂SO₄ | mittelbraun | ++ |
| 1,8-Bis-(2,5-diaminophenoxy)-3-6-dioxaoctan x 4HCl | gelb | ++ |
| 2-Methylamino-3-amino-6-methoxypyridin x 2HCl | dunkelbraun | ++ |
| 2-(2,5-Diaminophenyl)-ethanol x H₂SO₄ | graubraun | ++ |
| 2-Aminomethyl-4-aminophenol x 2HCl | braunorange | ++ |
| N,N-Bis-(2-hydroxyethyl)-p-phenylendiamin x H₂SO₄ | braunorange | ++(+) |
| 4,4'-Diaminodiphenylamin x H₂SO₄ | schwarz | +++ |
| 2,6-Dimethoxy-3,5-diaminopyridin x 2HCl | dunkelbraun | +++ |

**Tabelle 3**

| **Ausfärbungen mit 5-(N-Methylanilino)-2,4-pentadienal** | | |
|---|---|---|
| Komponente B | Färbenuance | Farbtiefe |
| - | gelb | ++ |
| 2,5-Diaminotoluol x H₂SO₄ | dunkelbraun | +++ |
| 2,4,5,6-Tetraaminopyrimidin x H₂SO₄ | orange | ++(+) |
| 1,8-Bis-(2,5-diaminophenoxy)-3-6-dioxaoctan x 4HCL | dunkelbraun | +++ |
| 2-Methylamino-3-amino-6-methoxypyridin x 2HCl | rotbraun | ++(+) |
| 2-(2,5-Diaminophenyl)-ethanol x H₂SO₄ | schwarzbraun | +++ |
| 2-Aminomethyl-4-aminophenol x 2HCl | rostrost | ++(+) |
| N,N-Bis-(2-hydroxyethyl)-p-phenylendiamin x H₂SO₄ | schwarz | +++ |
| 4,4'-Diaminodiphenylamin x H₂SO₄ | schwarz | +++ |
| 2,6-Dimethoxy-3,5-diaminopyridin x 2HCl | schwarzviolett | +++ |

**Tabelle 4**

| **Ausfärbungen mit N-(5-N'-Methylanilino-2,4-pentadienyliden-1-al)-N-methylanilinium-bromid** | | |
|---|---|---|
| Komponente B | Färbenuance | Farbtiefe |
| - | gelborange | ++(+) |
| 2,5-Diaminotoluol x H₂SO₄ | dunkelbraun | +++ |
| 2,4,5,6-Tetraaminopyrimidin x H₂SO₄ | gelborange | ++(+) |
| 1,8-Bis-(2,5-diaminophenoxy)-3-6-dioxaoctan x 4HCl | braunschwarz | +++ |
| 2-Methylamino-3-amino-6-methoxypyridin x 2HCl | dunkelbraun | +++ |
| 2-(2,5-Diaminophenyl)-ethanol x H₂SO₄ | braunviolett | +++ |
| 2-Aminomethyl-4-aminophenol x 2HCl | rostrot | ++(+) |
| N,N-Bis-(2-hydroxyethyl)-p-phenylendiamin x H₂SO₄ | schwarzviolett | +++ |
| 4,4'-Diaminodiphenylamin x H₂SO₄ | schwarz | +++ |

**Tabelle 5**

| **Ausfärbungen mit N-(5-Anilino-2,4-pentadienyliden)-anilin + HCl** | | |
|---|---|---|
| Komponente B | Färbenuance | Farbtiefe |
| - | rotorange | +++ |
| 2,5-Diaminotoluol x H₂SO₄ | grünschwarz | +++ |
| 2,4,5,6-Tetraaminopyrimidin x H₂SO₄ | orange | ++(+) |
| 1,8-Bis-(2,5-diaminophenoxy)-3-6-dioxaoctan x 4HCl | blauschwarz | +++ |
| 2-Methylamino-3-amino-6-methoxypyridin x 2HCl | braunschwarz | +++ |
| 2-(2,5-Diaminophenyl)-ethanol x H₂SO₄ | graugrün | ++(+) |
| 2-Aminomethyl-4-aminophenol x 2HCl | dunkelrot | +++ |
| N,N-Bis-(2-hydroxyethyl)-p-phenylendiamin x H₂SO₄ | graugrün | ++(+) |
| 4,4'-Diaminodiphenylamin x H₂SO₄ | braunschwarz | +++ |
| 2,6-Dimethoxy-3,5-diaminopyridin x 2HCl | braunschwarz | +++ |
| Indolin | kupfer | ++ |

**Tabelle 6**

| **Ausfärbungen mit N-[7-Chlor-2,4,6-hepta-trienyliden]-dimethylammoniumperchlorat** | | |
|---|---|---|
| Komponente B | Färbenuance | Farbtiefe |
| - | orangebraun | ++ |
| 2,5-Diaminotoluol x H₂SO₄ | schwarz | +++ |
| 2,4,5,6-Tetraaminopyrimidin x H₂SO₄ | dunkelrot | ++(+) |
| 1,8-Bis-(2,5-diaminophenoxy)-3-6-dioxaoctan x 4HCl | dunkelblaugrau | ++(+) |
| 2-Methylamino-3-amino-6-methoxypyridin x 2HCl | dunkelbraun | +++ |
| 2-(2,5-Diaminophenyl)-ethanol x H₂SO₄ | blauschwarz | +++ |
| 2-Aminomethyl-4-aminophenol x 2HCl | schwarzblau | +++ |
| N,N-Bis-(2-hydroxyethyl)-p-phenylendiamin x H₂SO₄ | blauschwarz | +++ |
| 4,4'-Diaminodiphenylamin x H₂SO₄ | braunschwarz | +++ |
| 2,6-Dimethoxy-3,5-diaminopyridin x 2HCl | schwarz | +++ |

**Tabelle 7**

| **Ausfärbungen mit Dimethyl-[7-dimethylamino-2,4,6-hepta-trienyliden]-dimethylammonium-perchlorat** | | |
|---|---|---|
| Komponente B | Färbenuance | Farbtiefe |
| - | dunkelrostrot | ++(+) |
| 2,5-Diaminotoluol x H₂SO₄ | violettschwarz | +++ |
| 2,4,5,6-Tetraaminopyrimidin x H₂SO₄ | rot | ++(+) |
| 1,8-Bis-(2,5-diaminophenoxy)-3-6-dioxaoctan x 4HCl | grauviolett | ++(+) |
| 2-Methylamino-3-amino-6-methoxypyridin x 2HCl | violettschwarz | +++ |
| 2-(2,5-Diaminophenyl)-ethanol x H₂SO₄ | dunkelviolett | +++ |
| 2-Aminomethyl-4-aminophenol x 2HCl | schwarz | +++ |
| N,N-Bis-(2-hydroxyethyl)-p-phenylendiamin x H₂SO₄ | schwarzviolett | +++ |
| 4,4'-Diaminodiphenylamin x H₂SO₄ | schwarz | +++ |
| 2,6-Dimethoxy-3,5-diaminopyridin x 2HCl | dunkelviolett | +++ |

## Patentansprüche

1. Mittel zum Färben von keratinhaltigen Fasern, insbesondere menschlichen Haaren, enthaltend als färbende Komponente mindestens ein Aminovinylaldehyd oder -keton oder ein Derivat davon an der Carbonylgruppe mit der Formel I oder deren physiologisch verträglichen Salze 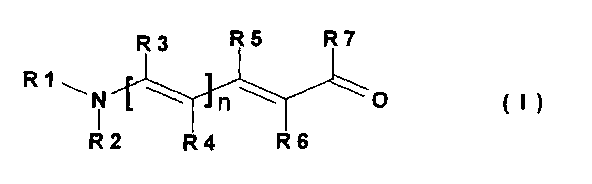
in der R¹ und R² unabhängig voneinander ein Wasserstoffatom, eine (C₁-C₄)-Alkylgruppe, Hydroxy-(C₁-C₄)-alkylgruppe, eine Phenylgruppe, die substituiert sein kann, oder gemeinsam mit dem N-Atom einen heterocyclischen Ring bilden, bedeuten,
R³, R⁴, R⁵, R⁶ und R⁷ für ein Wasserstoffatom, eine (C₁-C₄)-Alkylgruppe oder ein Halogenatom stehen, und
n 0, 1 oder 2 bedeutet,
wobei R⁶ und R⁷ sowie R⁴ und R⁶, wenn n gleich 1 ist, jeweils gemeinsam einen Ring bilden können.

2. Mittel nach Anspruch 1, **dadurch gekennzeichnet, daß** das Derivat an der Carbonylgruppe der Verbindungen mit der Formel I Iminoderivate sowie Ketale oder Acetale mit C₁-C₄-Alkylalkoholen sind.

3. Mittel nach Anspruch 1 oder 2, **dadurch gekennzeichnet, daß** als Aminovinylaldehyd oder -keton 3-Aminoacrolein, 3-Dimethylaminoacrolein, 4-Dimethylamino-3-buten-2-on, 5-Dimethylamino-2,4-pentadienal, 5-Diethylamino-2,4-pentadienal, 5-N-Methylanilino-2,4-pentadienal, 5-Pyrrolidino-2,4-pentadienal, 5-Piperidino-2,4-pentadienal, 5-Morpholino-2,4-pentadienal, 5-Dimethylamino-2-methyl-2,4-pentadienal, 5-Dimethylamino-3-methyl-2,4-pentadienal, 5-Dimethylamino-4-methyl-2,4-pentadienal, 5-Dimethylamino-3,5-dimethyl-2,4-pentadienal, 5-Dimethylamino-3-chlor-2,4-pentadienal, 6-Dimethylamino-3,5-hexadien-2-on 7-Dimethylamino-2,4,6-heptatrienal, 7-Morpholino-2,4,6-heptatrienal, 3-Dimethylaminomethylen-2-chlor-1-formyl-1-cyclohexen, 3-Dimethylaminomethylen-1-formyl-1-cyclopenten und deren physiologisch verträglichen Salze, insbesondere deren Alkalisalze, enthalten ist.

4. Mittel nach einem der Ansprüche 1 bis 3, **dadurch gekennzeichnet, daß** die Aminovinylaldehyde und/oder -ketone oder deren Derivate an der Carbonylgruppe mit der Formel I in einer Menge von 0,03 bis 65 mmol, insbesondere von 1 bis 40 mmol, bezogen auf 100 g des gesamten Färbemittels, enthalten sind.

5. Mittel nach einem der Ansprüche 1 bis 4, **dadurch gekennzeichnet, daß** es zusätzlich mindestens eine Verbindung mit primärer oder sekundärer Aminogruppe oder Hydroxygruppe, ausgewählt aus primären oder sekundären aliphatischen oder aromatischen Aminen, stickstoffhaltigen heterocyclischen Verbindungen, Aminosäuren, aus 2 bis 9 Aminosäuren aufgebauten Oligopeptiden und aromatischen Hydroxyverbindungen, und/oder mindestens eine CH-aktive Verbindung, enthält.

6. Mittel nach Anspruch 5, **dadurch gekennzeichnet, daß** die weitere Verbindung ausgewählt ist aus
primären oder sekundären Aminen aus der Gruppe, bestehend aus N-(2-Hydroxyethyl)-N-ethyl-, N-(2-Methoxyethyl-), 2,3-, 2,4-, 2,5-Dichlor-p-phenylendiamin, 2-Chlor-p-phenylendiamin, N,N-Bis-(2-hydroxyethyl)-p-phenylendiamin, 2,5-Dihydroxy-4-morpholinoanilin-dihydrobromid, 2-, 3-, 4-Aminophenol, o-, m-, p-Phenylendiamin, 2,4-Diaminophenoxyethanol, 2-(2,5-Diaminophenyl)-ethanol, 2,5-Diaminotoluol, -phenol, -phenethol, 4-Methylamino-, 3-Amino-4-(2 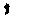 -hydroxyethyloxy)-, 3,4-Methylendiamino-, 3,4-Methylendioxyanilin, 3-Amino-2,4-dichlor-, 4-Methylamino-, 2-Methyl-5-amino-, 3-Methyl-4-amino-, 2-Methyl-5-(2-hydroxyethylamino)-, 2-Methyl-5-amino-4-chlor-, 6-Methyl-3-amino-2-chlor-, 5-(2-Hydroxyethylamino)-4-methoxy-2-methyl-, 4-Amino-2-aminomethyl-phenol, 4-Amino-2-hydroxymethyl-phenol, 1,3-Diamino-2,4-dimethoxybenzol, 2-, 3-, 4-Aminobenzoesäure, -phenylessigsäure, 2,3-, 2,4-, 2,5-, 3,4-, 3,5-Diaminobenzoesäure, 4-, 5-Aminosalicylsäure, 3-Amino-4-hydroxy-, 4-Amino-3-hydroxy-benzoesäure, 2-, 3-, 4-Aminobenzolsulfonsäure, 3-Amino-4-hydroxybenzolsulfonsäure, 4-Amino-3-hydroxynaphthalinsulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-, 1,2,4-Triaminobenzol, 1,2,4,5-Tetraaminobenzol-tetrahydrochlorid, 2,4,5-Triaminophenol-trihydrochlorid, Pentaaminobenzol-pentahydrochlorid, Hexaaminobenzol-hexahydrochlorid, 2,4,6-Triaminoresorcin-trihydrochlorid, 4,5-Diaminobrenzcatechinsulfat, 4,6-Diaminopyrogallol-dihydrochlorid, 3,5-Diamino-4-hydroxybrenzcatechin-sulfat, aromatische Aniline bzw. Phenole mit einem weiteren aromatischen Rest wie 4,4'-Diaminostilben-dihydrochlorid, 4,4'-Diaminostilben-2,2'-disulfonsäure, Na-Salz, 4,4 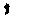 -Diaminodiphenylmethan, -sulfid, -sulfoxid, -amin, 4,4'-Diaminodiphenylamin-2-sulfonsäure, 4,4'-Diaminobenzophenon, -diphenylether, 3,3',4,4'-Tetraaminodiphenyl-tetrahydrochlorid, 3,3',4,4'-Tetraamino-benzophenon, 1,3-Bis-(2,4-diaminophenoxy)-propan-tetrahydrochlorid, 1,8-Bis-(2,5-diaminophenoxy)-3,6-dioxaoctan-tetrahydrochlorid, 1,3-Bis-(4-aminophenylamino)-propan, -2-propanol, 1,3-Bis-[N-(4-aminophenyl)-2-hydroxyethylamino]-2-propanol, N,N-Bis-[2-(4-aminophenoxy)-ethyl]methylamin-trihydrochlorid,
stickstoffhaltigen heterocyclischen Verbindungen ausgewählt aus der Gruppe, bestehend aus 2-, 3-, 4-Amino-, 2-Amino-3-hydroxy-, 2,6-Diamino-, 2,5-Diamino-, 2,3-Diamino-, 2-Dimethylamino-5-amino-, 3-Amino-2-methylamino-6-methoxy-, 2,3-Diamino-6-methoxy-, 3,5-Diamino-2,6-dimethoxy-, 2,4,5-Triamino-, 2,6-Dihydroxy-3,4-dimethylpyridin, 4,5,6-Triamino-, 2-Hydroxy-4,5,6-triamino-, 4-Hydroxy-2,5,6-triamino-, 2,4,5,6-Tetraamino-, 2-Methylamino-4,5,6-triamino-, 2,4-, 4,5-Diamino-, 2-Amino-4-methoxy-6-methyl-pyrimidin, 2,3,4-Trimethylpyrrol, 2,4-Dimethyl-3-ethyl-pyrrol, 3,5-Diaminopyrazol, -1,2,4-triazol, 3-Amino-, 3-Amino-5-hydroxypyrazol, 2-,3-, 8-Aminochinolin, 4-Amino-chinaldin, 2-, 6-Aminonicotinsäure, 5-Aminoisochinolin, 5-, 6-Aminoindazol, 5-, 7-Aminobenzimidazol, -benzothiazol, 2,5-Dihydroxy-4-morpholinoanilin sowie Indol- und Indolinderivaten, wie 4-, 5-, 6-, 7-Aminoindol, 4-, 5-, 6-, 7-Hydroxyindol, 5,6-Dihydroxyindol, 5,6-Dihydroxyindolin und 4-Hydroxyindolin, sowie jeweils aus den mit vorzugsweise anorganischen Säuren gebildeten physiologisch verträglichen Salzen dieser Verbindungen,
aromatischen Hydroxyverbindungen ausgewählt aus der Gruppe, bestehend aus 2-, 4-, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-, 3-, 4-Methoxy-, 3-Dimethylamino-, 2-(2-Hydroxyethyl)-, 3,4-Methylendioxyphenol, 2,4-, 3,4-Dihydroxybenzoesäure, -phenylessigsäure, Gallussäure, 2,4,6-Trihydroxybenzoesäure, -acetophenon, 2-, 4-Chlorresorcin, 1-Naphthol, 1,5-, 2,3-, 2,7-Dihydroxynaphthalin, 6-Dimethylamino-4-hydroxy-2-naphthalinsulfonsäure, 3,6-Dihydroxy-2,7-naphthalinsulfonsäure, und CH-aktiven Verbindungen ausgewählt aus der Gruppe, bestehend aus 1,2,3,3-Tetramethyl-3H-indoliniumiodid, 1,2,3,5-Tetramethylindolinium-p-toluolsulfonat, 1,2,3,5-Tetramethylindolinium-methansulfonat, 2,3-Dimethyl-benzothiazoliumiodid, 2,3-Dimethyl-benzothiazolium-p-toluolsulfonat, Rhodanin, Rhodanin-3-essigsäure, 1-Ethyl(Methyl)-2-chinaldiniumiodid, Barbitursäure, Thiobarbitursäure, 1,3-Dimethyl(ethyl)thiobarbitursäure, Oxindol, Cumaranon und 1-Methyl-3-phenyl-2-pyrazolinon.

7. Mittel nach Anspruch 6, **dadurch gekennzeichnet, daß** die weitere Verbindung ausgewählt ist aus der Gruppe bestehend aus N-(2-Hydroxyethyl)-N-ethyl-, 2-Chlor-p-phenylendiamin, N,N-Bis-(2-hydroxyethyl)-p-phenylendiamin, 4-Aminophenol, p-Phenylendiamin, 2-(2,5-Diaminophenyl)-ethanol, 2,5-Diaminotoluol, 3,4-Methylendioxyanilin, 3-Amino-2,4-dichlor-, 2-Methyl-5-amino-, 3-Methyl-4-amino-, 2-Methyl-5-(2-hydroxyethylamino)-, 2-Methyl-5-amino-4-chlor-, 6-Methyl-3-amino-2-chlor-, 2-Aminomethyl-4-aminophenol, 2-Hydroxymethyl-4-aminophenol 3,4-Methylendioxyphenol, 3,4-Diaminobenzoesäure, 2,5-Diamino-, 2-Dimethylamino-5-amino-, 3-Amino-2-methylamino-6-methoxy-, 2,3-Diamino-6-methoxy-, 3,5-Diamino-2,6-dimethoxy-, 2,6-Dihydroxy-3,4-dimethylpyridin, 2-Hydroxy-4,5,6-triamino-, 4-Hydroxy-2,5,6-triamino-, 2,4,5,6-Tetraamino-, 2-Methylamino-4,5,6-triamino-pyrimidin, 3,5-Diaminopyrazol, 3-Amino-5-hydroxypyrazol, 5,6-Dihydroxyindol und 5,6-Dihydroxyindolin sowie jeweils aus den vorzugsweise mit anorganischen Säuren gebildeten physiologisch verträglichen Salzen dieser Verbindungen.

8. Mittel nach einem der Ansprüche 1 bis 7, **dadurch gekennzeichnet, daß** es direkt ziehende Farbstoffe aus der Gruppe der Nitrophenylendiamine, Nitroaminophenole, Anthrachinone oder Indophenole vorzugtsweise in einer Menge von 0,01 bis 20 Gew.-%, bezogen auf das gesamte Färbemittel, enthält.

9. Mittel nach einem der Ansprüche 1 bis 8, **dadurch gekennzeichnet, daß** Ammonium- oder Metallsalze ausgewählt aus der Gruppe der Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Erdalkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, zuzugeben werden.

10. Mittel nach einem der Ansprüche 1 bis 9, **dadurch gekennzeichnet, daß** es Oxidationsmittel, insbesondere H₂O₂, in einer Menge von 0,01 bis 6 Gew.-%, bezogen auf die Anwendungslösung, enthält.

11. Mittel nach einem der Ansprüche 1 bis 10, **dadurch gekennzeichnet, daß** es anionische, zwitterionische oder nichtionische Tenside enthält.

12. Verwendung von Aminovinylaldehyden oder -ketonen oder deren Derivaten an der Carbonylgruppe mit der Formel I oder deren physiologisch verträglichen Salzen 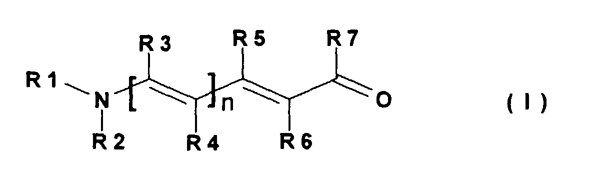
in der R¹ und R² unabhängig voneinander ein Wasserstoffatom, eine (C₁-C₄)-Alkylgruppe, Hydroxy-(C₁-C₄)-alkylgruppe, eine Phenylgruppe, die substituiert sein kann, oder gemeinsam mit dem N-Atom einen heterocyclischen Ring bilden, bedeuten,
R³, R⁴, R⁵, R⁶ und R⁷ für ein Wasserstoffatom, eine (C₁-C₄)-Alkylgruppe oder ein Halogenatom stehen, und
n 0, 1 oder 2 bedeutet,
wobei R⁶ und R⁷ sowie R⁴ und R⁶, wenn n gleich 1 ist, jeweils gemeinsam einen Ring bilden können
als eine färbende Komponente in Oxidationshaarfärbemitteln.

13. Verfahren zum Färben von keratinhaltigen Fasern, insbesondere menschlichen Haaren, worin ein Färbemittel, enthaltend
A) mindestens einen Aminovinylaldehyd oder -keton oder ein Derivat davon an der Carbonylgruppe mit der Formel I oder deren physiologisch verträglichen Salze 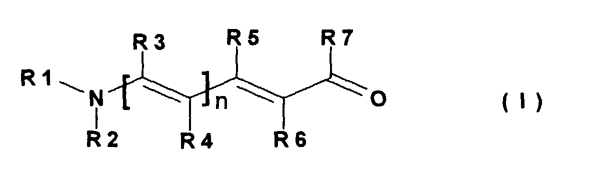
in der R¹ und R² unabhängig voneinander ein Wasserstoffatom, eine (C₁-C₄)-Alkylgruppe, Hydroxy-(C₁-C₄)-alkylgruppe, eine Phenylgruppe, die substituiert sein kann, oder gemeinsam mit dem N-Atom einen heterocyclischen Ring bilden, bedeuten,
R³, R⁴, R⁵, R⁶ und R⁷ für ein Wasserstoffatom, eine (C₁-C₄)-Alkylgruppe oder ein Halogenatom stehen und
n 0, 1 oder 2 bedeutet,
wobei R⁶ und R⁷ sowie R⁴ und R⁶, wenn n gleich 1 ist, jeweils gemeinsam einen Ring bilden können, und
B) mindestens eine Verbindung mit primärer oder sekundärer Aminogruppe oder Hydroxygruppe, ausgewählt aus primären oder sekundären aliphatischen oder aromatischen Aminen, stickstoffhaltigen heterocyclischen Verbindungen, Aminosäuren, aus 2 bis 9 Aminosäuren aufgebauten Oligopeptiden und aromatischen Hydroxyverbindungen, und/oder mindestens eine CH-aktive Verbindung,
sowie übliche kosmetische Inhaltsstoffe auf die keratinhaltigen Fasern aufgebracht, einige Zeit, üblicherweise ca. 30 Minuten, auf der Faser belassen und anschließend wieder ausgespült oder mit einem Shampoo ausgewaschen wird.
